(19) Europäisches Patentamt
European Patent Office
Office européen des brevets

(11) Veröffentlichungsnummer: **0 103 176**
**A2**

(12) **EUROPÄISCHE PATENTANMELDUNG**

(21) Anmeldenummer: **83107958.7**

(51) Int. Cl.³: **C 08 B 37/00**, C 12 P 19/06

(22) Anmeldetag: **11.08.83**

(30) Priorität: **14.08.82 DE 3230302**

(43) Veröffentlichungstag der Anmeldung: **21.03.84**
**Patentblatt 84/12**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **Fischer, Edgar, Dr., Assmannshäuser Weg 8, D-6000 Frankfurt am Main 71 (DE)**
Erfinder: **Schlingmann, Merten, Dr., Schneidhainer Strasse 32a, D-6240 Königstein/Taunus (DE)**

(54) **Isolierung mikrobieller Polysaccharide aus ihren Amin-Addukten.**

(57) Mikrobielle Polysaccharide lassen sich aus ihren Amin-Addukten durch Behandeln mit Ammoniak oder einem leichtflüchtigen Amin in Gegenwart einwertiger Alkanole mit 1 bis 3 Kohlenstoffatomen freisetzen. Vorteilhaft wird das Amin-Addukt in Form des feuchten Preßkuchens eingesetzt, wie es aus den Fermenterbrühen isoliert wird. Die freien Polysaccharide werden in praktisch quantitativer Ausbeute erhalten.

EP 0 103 176 A2

Isolierung mikrobieller Polysaccharide aus ihren Amin-Addukten

Die Erfindung betrifft ein Verfahren zur Isolierung von mikrobiellen Polysacchariden aus ihren Addukten mit Aminen.

Durch Fermentation hergestellte extrazelluläre mikrobielle Polysaccharide haben aufgrund ihrer hervorragenden Eigenschaften industrielle Anwendung als Verdicker, Gelier- oder Suspendiermittel, Schutzkolloide oder wasserbindende Mittel gefunden. Bedingt durch das Herstellungsverfahren sind diese Produkte recht teuer, wobei die bisher bekannten aufwendigen Isolierverfahren erheblich zu deren hohem Preis beitragen.

Aus der US-PS 3.928.316 ist es bekannt, das anionische Heteropolysaccharid, das durch Fermentation mit Hilfe des Bakteriums Xanthomonas campestris NRRL B-1459 erhalten wurde, als wasserunlösliches Salz eines primären langkettigen Amins aus den angesäuerten verdünnten Fermentationslösungen zu isolieren. Sofern eine Spaltung dieses Salzes beabsichtigt ist, erfolgt sie mit alkoholischer Kalilauge, wobei das Kaliumsalz erhalten wird, das aber noch aminhaltig ist.

In der veröffentlichten britischen Patentanmeldung 2.053.945 ist ein ähnliches Verfahren beschrieben, bei dem zur Fällung des Polysaccharids ein Polyamin verwendet wird. Sofern eine Isolierung des Amins aus dem Aminsalz beabsichtigt wird, wird hierfür die Behandlung des trockenen Salzes mit der Lösung einer starken Base in einer Flüssigkeit, die das freie saure Polysaccharid nicht löst wie wäßriges Methanol, angegeben.

Die Patentanmeldung ...... vom gleichen Anmeldetag (Deutsche Patentanmeldung P 32 30 303.3) betrifft ein Verfahren zur Abtrennung von mikrobiellen Polysacchariden aus ihren wäßrigen Lösungen durch Fällung im sauren Medium als Addukt

BAD ORIGINAL

mit einem langkettigen Alkylamin, das dadurch gekennzeichnet ist, daß ein Amin der Formel

$$NR^1R^2R^3$$

eingesetzt wird, in der $R^1$ Alkyl mit 10 - 20 Kohlenstoffatomen und $R^2$ und $R^3$, die gleich oder verschieden sind, Methyl oder Ethyl bedeuten.

Die bekannten Methoden zur Aufspaltung der Amin-Addukte verlaufen zum Teil recht unvollständig und führen zu salzhaltigen Polymeren. Somit werden weitere Reinigungsoperationen erforderlich, die nicht zuletzt wegen der Aufarbeitung der Abwässer aufwendig sind. Die Behandlung der Addukte mit starken Alkalien kann auch zu Schädigungen des Polymers führen.

Es wurde nun ein Verfahren zur Isolierung von mikrobiellen Polysacchariden aus ihren Amin-Addukten durch Behandeln mit alkalischen Mitteln in Gegenwart einwertiger Alkanole mit 1 bis 3 Kohlenstoffatomen gefunden, das dadurch gekennzeichnet ist, daß als alkalisches Mittel Ammoniak oder ein leichtflüchtiges Amin eingesetzt wird.

Im folgenden werden bevorzugte Ausgestaltungen der Erfindung näher erläutert:

Als Ausgangsmaterialien sind solche Addukte bevorzugt, die sich von Xanthan als mikrobiellem Polysaccharid und einem primären oder tertiären Fettalkylamin (mit zwei kurzkettigen Alkylresten) ableiten. Vorteilhaft werden diese Addukte im feuchten Zustand, beispielsweise als Preßkuchen, eingesetzt, wie sie bei der Isolierung der mikrobiellen Polysaccharide aus den Fermentationslösungen anfallen. Es entfällt so das Trocknen der Addukte, das nicht nur beachtliche Energiemengen erfordert, sondern auch das Löslichkeits- und Quellvermögen der Addukte beeinträchtigen

kann. Die geringen Wassermengen, die durch den Einsatz der feuchten Addukte eingebracht werden, stören beim erfindungsgemäßen Verfahren nicht.

Als Alkanole werden vorteilhaft die handelsüblichen technischen Qualitäten eingesetzt, wobei auch hierfür gilt, daß die geringen Wassermengen, die diese Produkte enthalten, das erfindungsgemäße Verfahren nicht beeinträchtigen. Größere Wassermengen sind jedoch nicht vorteilhaft, da die freien mikrobiellen Polysaccharide, insbesondere das Xanthan, in Wasser löslich, in den niederen Alkanolen jedoch unlöslich sind. Ein höherer Wassergehalt, im allgemeinen über 30 %, bezogen auf das Gewicht des Alkohols, könnte somit die Aufarbeitung erschweren. Wird deshalb mit dem feuchten Addukt oder mit dem Amin zu viel Wasser in das System eingebracht, wobei die zulässige Obergrenze vom Polysaccharid, dem Alkohol und dem Amin abhängt, so ist entsprechend mehr Alkohol einzusetzen.

Sofern Ammoniak als alkalisches Mittel dient, wird vorteilhaft eine Lösung im niederen Alkanol, insbesondere eine gesättigte Lösung, eingesetzt. Da Ammoniak ebenso wie primäre und sekundäre Amine mit reaktiven Gruppen, insbesondere Estergrupppen, der mikrobiellen Polysaccharide reagieren kann, arbeitet man hiermit zweckmäßig bei niederen bis mäßig erhöhten Temperaturen. Dadurch wird auch die Anwendung von Druck entbehrlich.

Bei Verwendung tertiärer Amine und beim Einsatz von Addukten tertiärer Amine kann hingegen bei erhöhter Temperatur gearbeitet werden, ohne daß es zu einer Produktschädigung kommt. Höhere Temperaturen verkürzen einerseits die Verfahrensdauer, andererseits bedingen Temperaturen in der Nähe des Siedepunkts des Amins bzw. des Alkohols aufwendigere Apparaturen. In der Praxis wird man deshalb eine den vorhandenen Apparaturen entsprechende zweckmäßige Temperatur einstellen, die anhand einfacher Vorversuche leicht zu ermitteln ist.

Unter leichtflüchtigen Aminen werden in erster Linie solche mit einem Siedepunkt unter 150°C bei Normaldruck verstanden. Amine mit höherem Siedepunkt sind weniger zweckmäßig, da diese Amine schwerer durch Destillation regenerierbar sind. Auch kann die Abtrennung von den aus den Addukten freigesetzten Aminen erschwert werden. Bevorzugt sind deshalb primäre Alkylamine mit einem Alkylrest bis zu 6 Kohlenstoffatomen, sekundäre Amine mit gleichen oder verschiedenen Alkylresten mit bis zu 4 Kohlenstoffatomen und tertiäre Amine mit gleichen oder verschiedenen Alkylresten mit bis zu 3 Kohlenstoffatomen. Besonders bevorzugt sind Trimethylamin, Triethylamin, Dimethylethylamin und Diethylmethylamin. Auch diese Amine werden vorteilhaft in Form ihrer Lösungen in dem eingesetzten Alkanol verwendet.

Die Auswahl des alkalischen Mittels ist auch von Einfluß auf die chemische Natur des Produkts: Aufgrund der hohen Flüchtigkeit kann auch ein Überschuß von Ammoniak beim Trocknen des freigesetzten Polysaccharids leicht und rasch entfernt werden. Wird ein aminfreies Produkt gewünscht, so wird man Ammoniak den Vorzug geben. Höher siedende Amine sind dann bevorzugt, wenn man das erfindungsgemäße Verfahren in Form eines Warmextraktionsverfahrens oder eines Destillationsverfahrens gestaltet, vor allem bei der kontinuierlichen Durchführung solcher Verfahren.

Das Gewichtsverhältnis von Alkohol zu Amin bzw. Ammoniak kann in weiten Grenzen schwanken, liegt aber vorteilhaft bei etwa 1 : 10 bis etwa 100 : 1. Da einige Addukte in Alkoholen unter Gelbildung stark solvatisieren, wird zweckmäßig von vornherein eine ausreichend konzentrierte alkoholische Lösung von Ammoniak oder Amin eingesetzt, da in diesen Lösungen die Solvatisierung unterdrückt wird. Auch das abgespaltene Polysaccharid solvatisiert nicht in diesen Lösungen und fällt als leicht abtrennbarer Niederschlag aus.

Da im heterogenen System gearbeitet wird, werden zweckmäßig Apparaturen eingesetzt, die eine Rühr-, Schneid-, Knet-, Scher- oder Mahlwirkung auf den Feststoff ausüben können.

Die mikrobiellen Polysaccharide fallen in einer leicht abtrennbaren Form an und liegen nach dem Trocknen weitgehend in der freien Säureform vor. Die Produkte zeichnen sich durch sehr günstige Viskositätseigenschaften aus.

Die aus Alkohol, dem eingesetzten und dem abgespaltenen Amin bestehende flüssige Phase läßt sich leicht destillativ aufarbeiten. Das aus dem Addukt abgespaltene Amin kann unmittelbar wieder zur Abtrennung mikrobieller Polysaccharide aus ihren Fermentationslösungen eingesetzt werden. Das übergehende Gemisch aus Alkohol und leichtflüchtigem Amin kann wieder in den Prozeß zurückgeführt werden. Die Wahl eines Amins mit einem geeigneten Siedepunkt erlaubt eine vollkontinuierliche Ausgestaltung des Verfahrens.

In den folgenden Beispielen beziehen sich Teile und Prozentangaben auf das Gewicht, sofern nichts anderes angegeben ist. Volumenteile stehen zu Gewichtsteilen im Verhältnis von l zu kg.

Zunächst wird die nicht-literaturbekannte Herstellung eines Addukts aus Xanthan und einem tertiären Fettalkylamin beschrieben (Deutsche Patentanmeldung P 32 30 303.3):

Als Produktionsstamm diente Xanthomonas campestris NRRL B-1459. Zur Vorkultur wurde eine Agarkultur in ein Glucose-Pepton-Medium überimpft und darin im Schüttelkolben bei 30°C bebrütet. Diese Kultur diente als Inoculum (3 %) für einen 10 Liter-Fermenter, dessen Nährmedium 3 - 5 % Glucose oder Saccharose, 0,15 - 0,25 % Cornsteep (Maisquellwasser), 0,1 bis 0,2 % Natriumnitrat, 0,1 % Dikaliumphosphat und 0,05 % Magnesiumsulfat-Hydrat

enthielt. Der beimpfte Fermenter wurde auf 28 °C gehalten und unter Rühren (400 U/min) mit 10 l Luft/min belüftet. Nach etwa 36 Stunden enthielt das Fermentationsmedium 18 - 20 g Xanthan pro Liter.

In 100 g einer Xanthanlösung mit einem Polysaccharidgehalt von 1,8 % wurden 0,85 g Talgfettalkyl-dimethylamin (C-Kettenverteilung im Talgfettalkylrest: etwa 5 % $C_{14}$; 30 % $C_{16}$; 65 % $C_{18}$) eingerührt. Nach Zugabe von 2,5 g 2 N Essigsäure gerann die erhaltene Dispersion und das Addukt schied sich in Form von zunächst stark gequollenen Fladen ab, die aber beim Nachrühren rasch desolvatisierten. Das Addukt wurde abfiltriert, mit entionisiertem Wasser nachgewaschen und durch Abpressen entwässert. Es wurden 6,2 g eines feuchten Preßkuchens erhalten, der 1,8 g Xanthan und 0,43 g Amin enthielt.

Beispiel 1

100 Teile eines feuchten Preßkuchens, bestehend aus dem Addukt von Talgfettalkylamin an Xanthan, das 32 % Xanthan enthielt, wurden in einem mit einem Schneidpropeller ausgerüsteten Mischer mit 6000 Teilen Methanol, das 1,5 % Ammoniak enthielt, desintegriert. Das dabei in Form von kurzen Fasern freigesetzte Xanthan wurde abfiltriert und mit Methanol nachgewaschen, bis das ablaufende Methanol neutral reagierte. Der Filterkuchen wurde getrocknet und zerkleinert. Das Xanthan wurde praktisch quantitativ (31,8 Teile) erhalten. Das Produkt war in entionisiertem Wasser vollkommen löslich. Die methanolischen Mutterlaugen waren ohne Zwischenaufarbeitung für mehrere Ansätze einsetzbar.

Beispiel 2

Es wurde wie in Beispiel 1 gearbeitet, nur wurde ein feuchter Preßkuchen eines Adduktes von Talgfettalkyldimethylamin und Xanthan eingesetzt. Das praktisch quantitativ erhaltene Xanthan löste sich vollständig in entionisiertem Wasser.

Beispiel 3

10 Teile eines feuchten Preßkuchens, bestehend aus dem Addukt von Lauryldimethylamin und Xanthan, das 36 % Xanthan enthielt, wurden in einem Mixer mit 150 Teilen einer Mischung von 2 Volumenteilen iso-Propanol und 1 Volumenteil Triethylamin desintegriert. Die entstandene Suspension wurde in einem Heißextraktor mit dem gleichen Gemisch aus iso-Propanol und Amin kontinuierlich extrahiert. Nach etwa 3 Stunden war die Spaltung beendet. Eine Probe des Extraktionsgutes war in entionisiertem Wasser vollständig löslich und zeigte nach dem Trocknen die für freies Xanthan typischen Eigenschaften.

Patentansprüche:

1. Verfahren zur Isolierung von mikrobiellen Polysacchariden aus ihren Amin-Addukten durch Behandeln mit alkalischen Mitteln in Gegenwart einwertiger Alkanole mit 1 bis 3 Kohlenstoffatomen, dadurch gekennzeichnet, daß als alkalisches Mittel Ammoniak oder ein leichtflüchtiges Amin eingesetzt wird.

2. Verfahren nach Anspruch 1, dadurch gekennzeichnet, daß das Ammoniak bzw. das Amin als Lösung in dem Alkanol eingesetzt wird.

3. Verfahren nach Anspruch 1 oder 2, dadurch gekennzeichnet, daß ein Amin mit einem Siedepunkt unter 150°C bei Normaldruck eingesetzt wird.

4. Verfahren nach einem oder mehreren der Ansprüche 1 bis 3, dadurch gekennzeichnet, daß als leichtflüchtiges Amin ein tertiäres Amin eingesetzt wird.

5. Verfahren nach einem oder mehreren der Ansprüche 1 bis 4, dadurch gekennzeichnet, daß das Gewichtsverhältnis von Alkohol zu Amin bzw. Ammoniak 1 : 10 bis 100 : 1 beträgt.

6. Verfahren nach einem oder mehreren der Ansprüche 1 bis 5, dadurch gekennzeichnet, daß das Amin-Addukt in Form eines feuchten Preßkuchens eingesetzt wird.

7. Verfahren nach einem oder mehreren der Ansprüche 1 bis 6, dadurch gekennzeichnet, daß das mikrobielle Polysaccharid Xanthan ist.

8. Verfahren nach einem oder mehreren der Ansprüche 1 bis 7, dadurch gekennzeichnet, daß das Amin im Addukt ein primäres Fettalkylamin ist.

9. Verfahren nach einem oder mehreren der Ansprüche 1 bis 7, dadurch gekennzeichnet, daß das Amin im Addukt ein tertiäres Fettalkylamin mit zwei kurzkettigen Alkylresten ist.

10. Verfahren nach einem oder mehreren der Ansprüche 1 bis 9, dadurch gekennzeichnet, daß ohne Druck bei einer Temperatur unterhalb des Siedepunktes des Reaktionsgemisches gearbeitet wird.